(19) Europäisches Patentamt
European Patent Office
Office européen des brevets

(11) Publication number: **0 307 767 B1**

(12) **EUROPEAN PATENT SPECIFICATION**

(45) Date of publication of patent specification: **21.07.93**

(51) Int. Cl.$^5$: **C07D 475/14**

(21) Application number: **88114546.0**

(22) Date of filing: **07.09.88**

(54) **Novel form of riboflavin.**

(30) Priority: **18.09.87 US 98436**

(43) Date of publication of application:
**22.03.89 Bulletin 89/12**

(45) Publication of the grant of the patent:
**21.07.93 Bulletin 93/29**

(84) Designated Contracting States:
**AT BE CH DE FR GB IT LI NL**

(56) References cited:
**EP-A- 0 020 959**
**EP-A- 0 161 548**
**US-A- 2 603 633**
**US-A- 2 797 215**
**US-A- 4 687 847**

(73) Proprietor: **F. HOFFMANN-LA ROCHE AG**
**Postfach 3255**
**CH-4002 Basel(CH)**

(72) Inventor: **Herena, Louis Edward**
**25 Howard Avenue**
**Eastchester, N.Y. 10709(US)**
Inventor: **Ramanarayanan, Kuttanchery Ananthanarayanan**
**1 Rustic Ridge B20**
**Little Falls, N.J. 07424(US)**

(74) Representative: **Cottong, Norbert A. et al**
**Grenzacherstrasse 124 Postfach 3255**
**CH-4002 Basel (CH)**

EP 0 307 767 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention is concerned with a process for producing a novel spherulitic form of riboflavin (vitamin $B_2$).

This process is characterized in that riboflavin is dissolved in a primary solvent, being a mineral acid, an organic acid or a mixture of these acids with each other and/or with water, and that a secondary solvent in which riboflavin is generally not soluble but which is miscible with the primary solvent, said secondary solvent being a $C_{1-4}$ - alkanol, a ketone, an organosulfoxide, a glycol, an ether, an organoamide, an organonitrile, a weak mineral acid, an organic acid, an organic base or water, is added at a temperature of from about 0°C to about 70°C to the obtained solution of riboflavin in the primary solvent, thereby precipitating the riboflavin in spherulitic form.

Riboflavin or vitamin $B_2$ is used extensively as an essential nutrient and sometimes colorant additive by the food and pharmaceutical industries. Typically all known crystal forms of riboflavin have the form of long yellow needles, and exhibit very poor handling characteristics and flow properties. These characteristics make it practically impossible to tablet or otherwise compact the compound in its pure form. Even in tablet granulations where riboflavin represents more than a small percentage of the mixture weight, the recalcitrant flow properties of the riboflavin disrupt the overall flow properties of the granula and inhibit uniform filling of the tablet die cavity resulting in unsatisfactory tablet to tablet weight distributions. To overcome this problem, manufacturers granulate (wet or dry) riboflavin with adjuvants to produce a product which exhibits acceptable flow and compression properties. These additional manufacturing procedures increase the cost and time for processing riboflavin. They also suffer from the disadvantage of having to include adjuvants with the riboflavin rather than being able to tablet pure riboflavin.

In addition, the undesirable handling and flow properties of riboflavin make it necessary to physically or mechanically predisperse and serially dilute the compound into a tablet granulation to assure acceptable tablet to tablet distribution of riboflavin in tablets containing a concentration of riboflavin equivalent to one Recommended Daily Allowance (RDA) (ca 2mg).

These drawbacks have now been eliminated by the process of the present invention.

In fact, the obtained product exhibits superior handling and flow properties and is directly compressible in tabletting applications. The spherulitic forms contrast with the needle form of riboflavin as presently available (i.e., prior art riboflavin), which exhibits very poor handling and flow properties and which must be coated and adjuvants added to be compressible.

U.S. Patent Specification 2,603 633 describes three different crystalline forms of riboflavin, designated therein as Types A,B and C and differing inter alia in their solubility in water and alcohol. Type C, with which this prior art is particularly concerned, is in the form of clusters of short wide needles or plates and is prepared by dissolving crude riboflavin in an aqueous alkali to give a riboflavin concentration of about 10 - 30 g/l, filtering the solution and then acidifying it with an acid such as hydrochloric, sulfuric, nitric, phosphoric or acetic acid, whereupon riboflavin immediately crystallizes out as Type C. The crystals can then be filtered off, washed and dried.

U.S. Patent Specification 2,797,215 describes a process for producing crystalline riboflavin by dissolving the crude material in an aqueous alkali hydroxide, ammonium hydroxide or aliphatic amine solution, acidifying the resulting solution containing about 5 - 30 g/l of riboflavin to obtain a riboflavin precipitate, and boiling the resultant acidified slurry until "Type A" crystals (fibrous needles) are formed. The resulting Type A crystals can then be filtered off, washed and dried.

U.S. Patent Specification 4,687,847 describes a process for producing very pure riboflavin from crude riboflavin by dissolving the crude material in an aqueous solution of alkali metal hydroxide, if necessary purifying the resulting alkaline solution of riboflavin by treatment with active carbon or with a filtration aid followed by filtration, or by extraction with an inert solvent which is insoluble or only slightly soluble in water, adding the alkaline riboflavin solution so obtained to water at about 90 - 100°C to which an acid has been added to give a pH of about 6.5 - 0.8 while maintaining the temperature at about 40-100°C, heating the reaction mixture at about 90-100°C for 10-80 minutes, optionally with stirring, and finally cooling the reaction mixture and isolating the riboflavin which has crystallized out.

European Patent Publication 0 020 959 describes a multi-step process of producing riboflavin from D-glucose. The crude riboflavin product can be purified by dissolution in aqueous hydrochloric acid, treatment of the acidic solution with hydrogen peroxide and precipitation of the riboflavin with water. Nothing is indicated about the crystalline form of the riboflavin so produced and purified.

Unlike the completely needle form of the prior art, standard riboflavin, the spherulitic riboflavin produced by the present invention has significally improved flow properties, which permit the novel riboflavin to be directly compressible into tablets. Moreover, certain spherulitic forms of the inventive riboflavin have bulk

densities which are higher than that of prior art riboflavin. This decreases storage space and increases ease of handling. The novel spherulitic forms of riboflavin are less electrostatic than the prior art riboflavin which results in improved handling and flow properties.

The inventive spherulitic riboflavin has been found to flow or generally move steadily and continuously through a channel or orifice. Its flow properties have been measured by the Agway funnel test as further described in Example 7 hereinbelow. Prior art pure riboflavin does not flow in the Agway funnel test. Not only does the riboflavin of the present invention flow in this test, which in itself is significant, it generally flows at from about 3 minutes per 100 grams to about 1 minute per 100 grams, and generally at less than about two minutes per 100 grams of spherulitic riboflavin. Agway funnel test results of this nature generally characterize products with excellent flow properties and which are suitable for use in tabletting equipment to produce unit dosage forms.

Advantageously, the bio-availability of spherulitic riboflavin is comparable with the prior art form of riboflavin. Accordingly, the novel spherulitic riboflavin exhibits the pharmaceutical characteristics of standard riboflavin, but with the above described advantages.

By pure riboflavin, one means riboflavin with a purity of 100% on a dry basis with a deviation of about 2 percent (i.e., 98-102% pure). It has been found that the spherulitic riboflavin produced in accordance with the present invention can be produced substantially free of impurities or other materials.

By spherulitic form, one means a configuration of riboflavin which has a dense core or nucleus and can have a repeated leafy, dendritic or needle-like structure on the periphery protruding in the radial outward direction.

As used herein, the term "lower" denotes residues and compounds having a maximum of 6, preferably a maximum of 4, carbon atoms.

The term "alkyl" connotes straight or branched chain saturated hydrocarbon groups of 1 to 20 carbon atoms. "Lower alkyl" denotes alkyl groups of 1 to 6 carbon atoms such as methyl, ethyl, n-propyl, isopropyl n-butyl, isobutyl and hexyl. Lower alkyl groups of 1-4 carbon atoms are preferred. "Alkoxy" denotes straight or branched chain alkoxy groups of 1 to 20 carbon atoms. Lower alkoxy connotes alkoxy groups having 1 to 6 carbon atoms such as methoxy, ethoxy, isopropoxy, propoxy, butoxy and isobutoxy. "Lower alkylene" refers to straight and branched chain alkylene groups having from 1 to 6 carbon atoms (e.g. methylene, ethylene and propylene). "Lower alkenyl" denotes straight or branched chain unsaturated univalent alkyl residues of 2 to 6 carbon atoms (ethenyl, isopropenyl, etc). "Lower alkanol" denotes alkanols of 1 to 6 carbon atoms, preferably 1-4 carbon atoms (e.g., methanol, ethanol, isopropanol, etc).

The term "cycloalkyl" denotes mononuclear and polynuclear saturated hydrocarbon groups of 1 to 20 carbon atoms. Suitable cycloaliphatic groups include cyclopentyl and cyclohexyl.

The term "alkanoyl" denotes straight-chain or branched-chain fatty acid residues derived from alkanecarboxylic acid moieties (e.g., formyl, acetyl, propionyl, etc.).

"Aryl" denotes mononuclear aromatic hydrocarbon groups such as phenyl and the like which can be unsubstituted or substituted in one or more positions with halogen, nitro, lower alkylenedioxy, lower alkyl or lower alkoxy. Aryl also denotes polynuclear aromatic groups such as napthyl, anthryl, phenanthryl, azulyl and the like which can be unsubstituted or substituted with one or more of the aforementioned substituents.

"Aralkyl" connotes an aryl lower alkyl group comprising aryl and lower alkyl moieties as defined hereinbefore. Examples are benzyl and alpha-lower alkyl substituted benzyls (e.g., cumyl).

The term "aryloxy" designates a moiety in which its aryl portion is defined as above (e.g., phenoxy, halo substituted phenoxy, nitro substituted phenoxy and lower alkyl substituted phenoxy).

The term "hetero atom" denotes oxygen, nitrogen or sulphur.

"Alkali metals" include lithium, sodium, potassium, cesium and rubidium. "Alkaline earth metals" include barium, magnesium, calcium and strontium.

"Halogen" denotes fluorine, chlorine, bromine and iodine. Halide connotes fluoride, chloride, bromide and iodide.

The term "organic acid" denotes carboxylic acids of the formula:

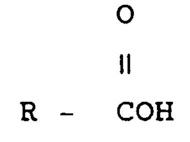

wherein R is hydrogen, lower alkyl, lower alkenyl, aryl, or aralkyl.
Preferably R is hydrogen, lower alkyl or phenyl. Examples of organic acids are formic acid, acetic acid, propionic acid, benzoic acid and the like.

The term "ketone" denotes a moiety of the formula:

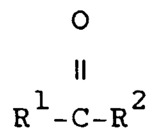

wherein $R^1$ and $R^2$ independently are lower alkyl, lower alkenyl, aryl or aralkyl.
Examples of ketones are acetone and methyl ethyl ketone.

An "ether" denotes a moiety of the formula:

$R^3$-O-$R^4$

wherein $R^3$ and $R^4$ individually are lower alkyl, lower alkenyl, aryl or aralkyl or $R^3$ and $R^4$ are taken together to form a 3 to 7 membered heterocyclic ring which can contain one or more hetero atoms in addition to the oxygen atom present in the above formula. Preferred ethers include tetrahydrofuran and dioxan.

The term "glycol" denotes conventional glycols and includes ethylene glycol, polyethylene glycol, propylene glycol and glycerol.

The term "organosulfoxide" denotes a compound of the formula:

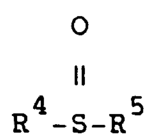

wherein $R^4$ and $R^5$ individually are lower alkyl, lower alkenyl, aryl or aralkyl.
A suitable organosulfoxide includes dimethylsulfoxide.

The term "organonitrile" denotes a compound of the formula:

$R^6$-C≡N

wherein $R^6$ is lower alkyl, lower alkenyl, aryl or aralkyl.

The term primary, secondary and tertiary amine denotes amines of the formula:

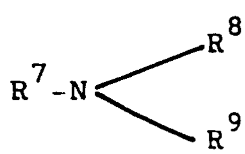

wherein $R^7$-$R^9$ individually are hydrogen, lower alkyl, lower alkenyl, aryl or aralkyl.

The term "organic base" denotes conventional organic bases such as primary, secondary and tertiary amines as well as aqueous basic solutions such as aqueous urea solutions and aqueous ammonium sulfate solutions.

The term "organoamide" denotes primary, secondary and tertiary amides of the formula

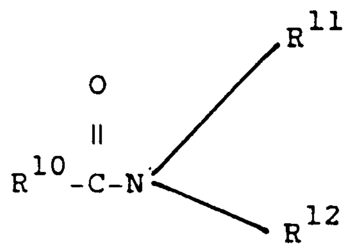

wherein $R^{10}$-$R^{12}$ individually are hydrogen, lower alkyl, lower alkenyl, aryl or aralkyl.

Examples of amides include formamide and dimethylformamide.

The inventive process is based on a two step procedure involving two solvents: a primary solvent, as more closely specified above, in which the solute (riboflavin) is soluble, and a secondary solvent, as also more closely specified above, in which the solute is relatively insoluble but which is miscible with the primary solvent.

In the first step of the inventive process, technical grade or pure prior art riboflavin (i.e., needle form) is dissolved in the primary solvent. Suitable primary solvents include strong and weak mineral acids and strong and weak organic acids. Examples include hydrochloric acid, nitric acid, sulfuric acid, phosphoric acid, hydrobromic acid, methane sulfonic acid, formic acid, and acetic acid. Mixtures of these acids with each other and/or with water are within the skill of the art and also are suitable.

The preferred primary solvent is a strong mineral acid, especially hydrochloric acid.

In general, it is within the skill of an artisan to select conventional primary solvents, which will dissolve riboflavin in various concentrations of the primary solvent with or without the addition of water. Preferably, riboflavin is completely dissolved in the primary solvent with the concentration of riboflavin varying from about 8% to about 60% by weight depending upon the solubility of riboflavin in the primary solvent. The concentration of riboflavin may be increased to more than 60% but this is generally not practical at room temperature (about 23°C) because of the high viscosity encountered with such solutions. The rate of addition of vitamin $B_2$ to the primary solvent is not critical and within the skill of an artisan to adjust. The dissolution of riboflavin in accordance with the first step of the invention procedure, however, is slightly exothermic. When using strong oxidizing reagents, such as nitric acid, the above dissolution process step could be highly exothermic and thus the dissolution should be carefully monitored.

In the first process step, temperature and pressure are not critical but room temperature and atmospheric pressure are preferred. To increase yield, it also is preferred that the dissolution be as complete as possible.

In accordance with a preferred embodiment of the present invention, the solution of riboflavin and the primary solvent is filtered to separate out any solids such as undissolved riboflavin and impurities. This filtration step, however, is not necessary and can be eliminated depending upon the specifications (particularly the purity) for the riboflavin to be manufactured.

In the second step of the process, a secondary solvent then is mixed with or added to the solution formed in the first step of the process so as to precipitate the spherulitic riboflavin. The secondary solvent is selected so that it is miscible with the primary solvent but has low solubility for riboflavin. The solubility of riboflavin in the selected secondary solvent will depend on the system chosen (e.g., primary solvent, temperature, pressure, etc.). The acceptable value for solubility, thus, will vary from solvent system to solvent system. However, the solubility of riboflavin in the secondary solvent should generally be about 1 mg/liter to 1400 mg/liter, preferably about 30 mg/liter to about 100 mg/liter. Since many of the primary solvents suitable for use in the present invention are a mixture of a mineral acid with water or an organic acid with water, preferably the secondary solvent should be water-miscible. This is required should the primary solvent contain water.

Suitable secondary solvents include branched or straight chain alkanols of 1 to 4 carbon atoms (e.g. methanol, ethanol, isopropanol and the like), ketones (e.g. acetone and methyl ethyl ketone), organosulfoxides (e.g. dimethyl sulfoxide (DMSO)), glycols with molecular weights of about 60 to about 420 g/gmol (e.g. ethylene glycol, diethylene glycol, polyethylene glycol, propylene glycol, and glycerol), ethers (e.g. tetrahydrofuran (THF) and dioxan), organoamides (e.g., formamide and dimethylformamide (DMF)) organonitriles (e.g. acetonitrile), weak mineral acids (aqueous boric acid solution and aqueous ammonium sulfate solution), organic acids, organic bases (e.g. primary, secondary, and tertiary amines, and aqueous

urea solution) and water. Preferably the secondary solvent is a $C_{1-4}$ alkanol or water, especially methanol, ethanol or isopropanol.

The selection of an appropriate secondary solvent for use in conjunction with the selected primary solvent is within the skill of an artisan knowing the description contained in this specification. Certain secondary solvents achieve better yield or become suitable by the addition of water thereto. For example, it is found that the addition of water to the following secondary solvents facilitates the formation of spherulitic riboflavin: acetone, dimethylsulfoxide, tetrahydrofuran, glycols, dioxan, and dimethylformamide. Moreover, it is found that water, per se, can be used as a suitable secondary solvent depending upon the primary solvent selected. Illustratively, with the selection of sulfuric acid, formic acid, hydrochloric acid or hydrobromic acid as the primary solvent, water, per se, is found suitable as the secondary solvent.

A preferred combination of primary and secondary solvents involves the use of hydrochloric acid as the primary solvent and of methanol, ethanol or water as the secondary solvent.

As mentioned above, in the second step of the process the temperature at which the secondary solvent is added ranges from about 0 °C to about 70 °C. The preferred temperature range is from about 20 °C to about 35 °C. The mixing of the secondary solvent with the solution produced by the first step could result in a temperature increase and this should be monitored. Preferably the reaction is carried out at atmospheric pressure. By altering the temperature or pressure selected, one can control the type of spherulites produced and, for example, can create individual spherulites or precipitate agglomerates or aggregates of spherulites such as two or more spherulites attached together.

To assist in achieving the inventive spherulitic forms of riboflavin, it is desirable to obtain a highly supersaturated solution of riboflavin in the second step of the process. This is accomplished by controlled batchwise or continuous mixing of the secondary solvent with the primary riboflavin solution of the first process step at the rates of addition described hereinbelow. The sequence of mixing the primary solution and the secondary solvent is not critical. In batch processing, the secondary solvent could be added to the primary solution or vice versa. In continuous processing the two components could be introduced together simultaneously.

The actual rate of addition (mixing) of the secondary solvent varies with the system (primary and secondary solvent, and the quantity of material to be processed, etc) selected. The rate of addition generally varies from about 1 g/min to about 1,600 kg/min, preferably about 100 g/min to about 800 kg/min. Moreover, the time of addition of the secondary solvent ranges generally from 5 seconds to about 30 minutes and preferably ranges from about 1 minute to about 20 minutes. Advantageously, the rate of addition of secondary solvent should be as high as possible (and thus the time of addition as low as possible) to obtain optimum size of the resulting spherulites.

More particularly, the exact size and configuration of the resulting spherulites can be modified by changing, inter alia, the rate of addition of the secondary solvent to the riboflavin solution or vice versa. A fast rate of addition tends to reduce the formation of needles from the nucleate. The spherulitic configuration can be modified to that of a relatively thicker or thinner central nucleate having radiating therefrom leaves, needles or spikes generally by changing the rate of addition. Preferably, the dendritic growth should be kept as small as possible and the the central nucleate as large as possible. This is accomplished by a high rate of addition. Other factors which impact upon the spherulitic growth include temperature of precipitation, agitation speed and drying and handling (i.e., pulverizing, sieving and the like) techniques.

During the second process step, it is advantageous to agitate the solution to achieve intermixing of the secondary solvent with the riboflavin solution of the first process step, but the speed of agitation should be at a moderate rate so as not to disintegrate the precipitating spherulites. An agitation rate of about 50 to about 200 revolutions per minute, depending upon the primary and secondary solvents selected and size of the equipment used, is suitable.

With the supersaturated state achieved by the second step of the process as described above, spherulitic riboflavin will precipitate from the solution.

Advantageously, the resulting slurry of precipitated riboflavin then is filtered, washed with a secondary solvent and dried to achieve pure spherulitic riboflavin. In a preferred embodiment, the slurry is filtered by conventional techniques such as centrifugation, and the resulting cake of riboflavin is washed with the secondary solvent previously selected, although other solvents can be utilized. It is preferred that water alone not be utilized in the washing because the resulting riboflavin becomes paste-like. The drying step is conducted in accordance with conventional drying techniques. Preferably, one uses those dryers which would not be destructive to the resulting spherulitic configurations, such as tray dryers or vibratory fluid bed dryers. The flow properties of the resulting spherulite forms of riboflavin, to an extent, depend upon the degree of drying of the inventive riboflavin. The resulting riboflavin can be dried to any degree of dryness which is desired such as for example 50 percent by weight on a dry basis. Preferably, but without intending

to limit the scope of the invention, the inventive riboflavin is dried to about less than 1.5 percent, preferably about 0.5 percent by weight of residual solvent(s) and most preferably about 0.1 percent by weight. Moreover, the drying process could cause an abrasion of at least a part of the spherulite surface thus reducing or eliminating the dendritic layer and leaving the dense core. All such process variations and resulting spherulite configurations are contemplated by the invention.

The spherulites obtained in accordance with the present invention are part crystalline in nature and part amorphous. The amorphous characteristics of the resulting spherulites can be examined by infrared spectrocopy, differential scanning calorimetry or x-ray diffraction.

The resulting spherulitic riboflavin can be directly tableted into unit dosage forms with or without adjuvants by conventional tableting techniques. It, however, is preferred to use tablet dye lubricants to avoid damage to the dyes.

While the invention has been described in conjunction with certain embodiments, it is understood that various modifications and changes may be made without departing from the spirit and scope of the invention. Illustratively, the spherulitic riboflavin can be made with impurities or free from impurities. Adjuvants generally recognized as safe (GRAS) and/or other active ingredients such as other vitamins can be combined and tableted with the spherulitic riboflavin. It can also be dried to varying degrees of dryness. The configuration of the spherulite such as the core or outer layer can be modified. Thus, the outer layer also can have a leaf-like structure or a more needle-like structure. This outer layer also can have various thicknesses or can be abraded to expose the core. All these and other modifications as mentioned in the specification or within the skill of an artisan are contemplated.

The following examples further illustrate the invention. All temperatures are in degrees centrigrade, and percentages are given by weight.

Example 1

Primary solvent: methanesulfonic acid (MSA)
Secondary solvent: 100% ethanol

150 g of 95.6% pure riboflavin ($B_2$)at 24.5 ° C were dissolved in 600 g of 70% methanesulfonic acid at 23.5 ° C, resulting in a solution at 27 ° C. This solution was stirred with a magnetic stirrer at moderate speed (about 60 rpm) in a 4000-ml beaker for 35 minutes, at which point the temperature dropped to 25.5 ° C. The solution was filtered and divided into two batches. To each of two batches of 360 g of the above solution in 4000-ml beakers, 1800 g of 100% ethanol at 24.0 ° C was added in 7 seconds. The $B_2$/MSA solution temperature rose from 24 to 29 ° C from beginning to end of ethanol addition. The induction time was approximately 300 seconds. The solution was left to precipitate for 70 minutes, at which time it was filtered and the particles washed with 4000 g of 100% ethanol. The wet cake weighed 415 g before drying in an oven at 100 ° C for 12 hours. The dried cake weighed 112 g.

Example 2

Primary solvent: hydrochloric acid
Secondary solvent: 100% ethanol

120 g of 95.6% pure riboflavin at 24.5 ° C were dissolved in 334 g of 37% hydrochloric acid (HCl) at 23 ° C, resulting in a solution at 29.5 ° C. This solution was stirred in a 4000-ml beaker for 30 minutes, at which point the temperature dropped to 27.5 ° C. The solution was then filtered. To 442 g of the filtered $B_2$/HCl solution in a 4000-ml beaker, 1100 g of 100% ethanol at 23.5 ° C was added in 11 seconds. The $B_2$/HCl solution temperature rose from 25 to 37 ° C at the end of ethanol addition. The induction time was approximately 80 seconds. The solution was left to precipitate for 20 minutes, at which time it was filtered and the particles washed with 2000 g of 100% ethanol. The wet cake was dried in an oven at 100 ° C for 12 hours. The dried cake weighed 106 g.

Example 3

Primary solvent: nitric acid
Secondary solvent: 30% glycerol, 70% water

150 g of 95.6% pure riboflavin at 23.5°C were dissolved in 450 g of 70.6% nitric acid at 23.5°C, resulting in a solution at 32°C. This solution was stirred in a 4000-ml beaker for 15 minutes, at which point the temperature dropped to 30.5°C. The solution was filtered. To 587 g of the above filtered solution in a 4,000-ml beaker, 3000 g of 30% glycerol at 24.0°C was added in 17 seconds. The temperature of the riboflavin in nitric acid solution rose from 28 to 30.5°C. The solution was left to precipitate for 20 minutes, at which time it was filtered and the particles washed with 3000 g of solvent-grade acetone. The wet cake weighed 233 g prior to drying for 12 hours in an oven at 100°C. The dry cake weighed 109 g.

Example 4

Primary solvent: formic acid
Secondary solvent: 100% methanol

258 g of 95.6% pure riboflavin at 19°C were dissolved in 3097 g of 99% formic acid at 19°C, resulting in a solution of 21°C. This solution was stirred in a 4000-ml beaker for 30 minutes, at which point the temperature dropped to 24.5°C. The solution was filtered. A total of 2500 g of the above solution in 4000-ml beakers was precipitated in 500 g batches using 2500 g of 100% methanol at 24.0°C each. The average addition time was 25 seconds. The temperature of the riboflavin-formic acid solution rose from 24 to 25°C from beginning to end of methanol addition. The average induction time was approximately 139 seconds. The resulting solutions were left to precipitate for about 25 minutes, at which time they were filtered and each of the batches washed with 4 liters of solvent-grade methanol. The combined wet cake weighed 409 g before drying in an oven at 100°C for 12 hours. The dry cake weighed 118 g.

Example 5

In an analagous manner to that described in Example 2, the following primary and secondary solvents have been utilized in accordance with the present invention to obtain spherulitic riboflavin. The percentages denote the percentage by weight of the mixture of the two primary solvents. The remaining percentage of the mixture is water. Should only one primary solvent be shown then the percentage denotes the concentration of the primary solvent in water.

TABLE 1

| Primary Solvent(s) | Secondary Solvent |
|---|---|
| acetic and hydrochloric acid (50%/18%) | acetone |
| acetic and hydrochloric acid (50%/18%) | diethylene glycol |
| acetic and hydrochloric acid (50%/18%) | dioxan |
| acetic and hydrochloric acid (50%/18%) | dimethyl sulfoxide |
| acetic and hydrochloric acid (50%/18%) | ethylene glycol |
| acetic and hydrochloric acid (50%/18%) | ethanol |
| acetic and hydrochloric acid (50%/18%) | glycerol |
| acetic and hydrochloric acid (50%/18%) | methanol |
| acetic and hydrochloric acid (50%/18%) | polyethylene glycol 40 |
| acetic and hydrochloric acid (50%/18%) | tetrahydrofuran |
| acetic and hydrochloric acid (50%/18%) | water |
| formic acid (99%) | acetone |
| formic acid (99%) | ethanol |
| formic acid (99%) | methanol |
| formic acid (99%) | water |
| hydrobromic acid (48%) | ethanol |
| hydrobromic acid (48%) | water |
| hydrochloric acid (36%) | acetone |
| hydrochloric acid (36%) | acetonitrile |
| hydrochloric acid (36%) | diethylene glycol |
| hydrochloric acid (36%) | dioxan |
| hydrochloric acid (36%) | dimethylformamide |
| hydrochloric acid (36%) | dimethyl sulfoxide |

| | |
|---|---|
| hydrochloric acid (36%) | ethylene glycol |
| hydrochloric acid (36%) | ethanol |
| hydrochloric acid (36%) | formamide |
| hydrochloric acid (36%) | glycerol |
| hydrochloric acid (36%) | isopropyl alcohol |
| hydrochloric acid (36%) | methyl ethyl ketone |
| hydrochloric acid (36%) | methanol |
| hydrochloric acid (36%) | polyethylene glycol 40 |
| hydrochloric acid (36%) | propylene glycol |
| hydrochloric acid (36%) | tetrahydrofuran |
| hydrochloric acid (36%) | water |
| hydrochloric acid (36%) | aqueous ammonium sulfate |
| hydrochloric acid (36%) | aqueous boric acid |
| hydrochloric acid (36%) | aqueous urea |
| methane sulfonic acid (70%) | ethanol |
| methane sulfonic acid (70%) | water |
| nitric acid (71%) | acetone |
| nitric acid (71%) | diethylene glycol |
| nitric acid (71%) | dioxane |
| nitric acid (71%) | dimethyl sulfoxide |
| nitric acid (71%) | ethylene glycol |
| nitric acid (71%) | ethanol |
| nitric acid (71%) | glycerol |
| nitric acid (71%) | methanol |
| nitric acid (71%) | tetrahydrofuran |
| nitric acid (71%) | water |
| phosphoric acid (85.4%) | ethanol |
| phosphoric acid (85.4%) | water |
| sulfuric acid (32.5%) | ethanol |
| sulfuric acid (32.5%) | water |

Example 6

Primary Solvent: Conc. hydrochloric acid
Secondary Solvent: 95% w/w ethanol

50 kg. of 95.6% riboflavin was added to a 300 gal. (1 gal. = 3.785332 l) glass-lined kettle, which contained 135 kg. of concentrated hydrochloric acid (37%). The reactor was agitated for 30 minutes to dissolve all of the riboflavin. The dissolved riboflavin solution, referred to as $B_2$/HCl solution, was then filtered by pumping through a cartridge filter and into a glass-lined precipitator. A feed tank was previously loaded with 480 kg. of 95% ethanol (5% water) and maintained at 20 to 25°C. The contents of the feed tank was transferred to the precipitator by gravity. The precipitator was agitated at 95 rpm. The time of addition was 19 minutes. The contents of precipitator was held for 2 hr. and then centrifuged and washed with 405 kg. of 95% chilled ethanol at 0°C. The resulting riboflavin cake (51% wet) was dried in a tray dryer at 40°C for 65.5 hr. 45.8 kg. of pure riboflavin was recovered, at a purity of 99.05% with a loss on drying of 0.9% resulting in a yield of 94.5%. The dried material was delumped and sieved through a 30-mesh screen to obtain final product.

Example 7

Agway Funnel Flow Test

The Agway funnel flow test is a technique for examining flow properties. The funnel is glass (thickness about 0.254 cm) and has a conical configuration, one opening (orifice) having a 1.11125 cm I.D. (inside diameter), the other having a 8.89 cm I.D. It is 20.32 cm long and the angle between its longitudinal axis and the wall is 10 degrees.

In accordance with the procedure the funnel is placed in a suitable ring stand with the orifice of the funnel located approximately six inches from a table top. 100 g of test material are weighed and poured into the above described funnel. Any loss of material from the funnel orifice is prevented by covering the orifice with a finger or plate. The material is permitted to flow from the funnel by removal of the finger and if needed lightly tapping the outside of funnel side wall with a finger or other rod like implement. The time required to empty the funnel is noted. The procedure is repeated at least three times, taking an average and recording it as the flow time.

Analysis of the flow properties is based on the flow time recorded, the visual flow behavior, and the number of taps to the funnel side wall necessary to initiate or maintain flow. Poor flow behavior patterns (e.g. no flow on one of the runs, considerable taps, long flow time, and erratic flow) does not automatically imply the material will fail to uniformly fill tablet dyes. Flow or non-flow through the Agway funnel serves as a useful vehicle in measuring the relative flowability of a product and for predicting potential flow problems in actual tabletting applications.

Example 8

Spherulitic Riboflavin

Listed below is a table of primary and secondary solvents, the yields achieved, the Agway funnel times and the bulk density of riboflavin in accordance with the present invention:

TABLE 2

| Example | Primary Solvent | Secondary Solvent | Agway Funnel Flow Time Seconds/100 g | Bulk Density, g/ml |
|---|---|---|---|---|
| 1 | Methane sulfonic acid | Ethanol | 70 | 0.16-0.23 |
| 2 | HCl | Ethanol | 95 | 0.33-0.45 |
| 3 | $HNO_3$ | Glycerol | 45 | 0.48-0.57 |
| 4 | Formic acid | Methanol | 18 | 0.43-0.60 |
| 6 | HCl | Ethanol | 86 | 0.27-0.33 |

## Claims

1. Process for producing riboflavin in a crystalline form, characterized in that riboflavin is dissolved in a primary solvent, being a mineral acid, an organic acid or a mixture of these acids with each other and/or with water, and that a secondary solvent in which riboflavin is generally not soluble but which is miscible with the primary solvent, said secondary solvent being a $C_{1-4}$-alkanol, a ketone, an organosulfoxide, a glycol, an ether, an organoamide, an organonitrile, a weak mineral acid, an organic acid, an organic base or water, is added at a temperature of from about 0 °C to about 70 °C to the obtained solution of riboflavin in the primary solvent, thereby precipitating the riboflavin in spherulitic form.

2. Process according to claim 1, characterized in that the primary solvent is a strong mineral acid.

3. Process according to claim 2, characterized in that the primary solvent is hydrochloric acid.

4. Process according to any one of claims 1 to 3, characterized in that the secondary solvent is a $C_{1-4}$ - alkanol or water.

5. Process according to claim 4, characterized in that the secondary solvent is methanol, ethanol or isopropanol.

6. Process according to any one of claims 1 to 5, characterized in that the primary solvent is hydrochloric acid and the secondary solvent is methanol, ethanol or water.

7. Process according to any one of claims 1 to 6, characterized in that the secondary solvent is added at a temperature of from about 20 °C to about 35 °C.

8. Process according to any one of claims 1 to 7, characterized in that the rate of addition of the secondary solvent to the solution is from about 1 g/min. to about 1600 kg/min.

9. Process according to claim 8, characterized in that the rate of addition of the secondary solvent to the solution is from about 100 g/min. to about 800 kg/min.

10. Process according to any one of claims 1 to 9, characterized in that the time of addition of the secondary solvent is from about 5 seconds to about 30 minutes.

11. Process according to claim 10, characterized in that the time of addition of the secondary solvent is from about 1 minute to about 20 minutes.

## Patentansprüche

1. Verfahren zur Herstellung von Riboflavin in kristalliner Form, dadurch gekennzeichnet, dass man Riboflavin in einem ersten Lösungsmittel, das eine Mineralsäure, eine organische Säure oder ein Gemisch dieser Säuren untereinander und/oder mit Wasser ist, löst, ein zweites Lösungsmittel zugibt, in welchem Riboflavin nicht allgemein löslich ist, welches jedoch mit dem ersten Lösungsmittel mischbar ist, wobei besagtes zweites Lösungsmittel ein $C_{1-4}$-Alkanol, ein Keton, ein Organosulfoxid, ein Glykol, ein Aether, ein Organoamid, ein Organonitril, eine schwache Mineralsäure, eine organische Säure, eine organische Base oder Wasser ist, und zwar bei einer Temperatur von etwa 0 °C bis etwa 70 °C zur erhaltenen Lösung des Riboflavins im ersten Lösungsmittel, so dass das Riboflavin in Form von Kügelchen ausfällt.

2. Verfahren nach Anspruch 1, dadurch gekennzeichnet, dass das erste Lösungsmittel eine starke Mineralsäure ist.

3. Verfahren nach Anspruch 2, dadurch gekennzeichnet, dass das erste Lösungsmittel Salzsäure ist.

4. Verfahren nach einem der Ansprüche 1 bis 3, dadurch gekennzeichnet, dass das zweite Lösungsmittel ein $C_{1-4}$-Alkanol oder Wasser ist.

5. Verfahren nach Anspruch 4, dadurch gekennzeichnet, dass das zweite Lösungsmittel Methanol, Aethanol oder Isopropanol ist.

6. Verfahren nach einem der Ansprüche 1 bis 5, dadurch gekennzeichnet, dass das erste Lösungsmittel Salzsäure und das zweite Lösungsmittel Methanol, Aethanol oder Wasser ist.

7. Verfahren nach einem der Ansprüche 1 bis 6, dadurch gekennzeichnet, dass das zweite Lösungsmittel bei einer Temperatur von etwa 20 °C bis etwa 35 °C zugegeben wird.

8. Verfahren nach einem der Ansprüche 1 bis 7, dadurch gekennzeichnet, dass die Rate der Zugabe des zweiten Lösungsmittels zur Lösung von etwa 1 g/Min. bis etwa 1600 kg/Min. beträgt.

9. Verfahren nach Anspruch 8, dadurch gekennzeichnet, dass die Rate der Zugabe des zweiten Lösungsmittels zur Lösung von etwa 100g/Min. bis etwa 800 kg/Min. beträgt.

10. Verfahren nach einem der Ansprüche 1 bis 9, dadurch gekennzeichnet, dass die Dauer der Zugabe des zweiten Lösungsmittels von etwa 5 Sekunden bis etwa 30 Minuten beträgt.

11. Verfahren nach Anspruch 10, dadurch gekennzeichnet, dass die Dauer der Zugabe des zweiten Lösungsmittels von etwa 1 Minute bis etwa 20 Minuten beträgt.

**Revendications**

1. Procédé de production de riboflavine sous forme cristalline, caractérisé en ce que l'on dissout la riboflavine dans un solvant primaire, qui est un acide minéral, un acide organique ou un mélange de ces acides l'un avec l'autre et/ou avec de l'eau, et en ce que l'on ajoute un solvant secondaire, dans lequel la riboflavine n'est généralement pas soluble, mais qui est miscible avec le solvant primaire, ledit solvant secondaire étant un alcanol en $C_1$-$C_4$, une cétone, un organosulfoxyde, un glycol, un éther, un organoamide, un organonitrile, un acide minéral faible, un acide organique, une base organique ou de l'eau, à une température d'environ 0 °C à environ 70 °C, à la solution de riboflavine dans le solvant primaire obtenue, ce qui précipite la riboflavine sous forme de sphérulite.

2. Procédé selon la revendication 1, caractérisé en ce que le solvant primaire est un acide minéral fort.

3. Procédé selon la revendication 2, caractérisé en ce que le solvant primaire est de l'acide chlorhydrique.

4. Procédé selon l'une quelconque des revendications 1 à 3, caractérisé en ce que le solvant secondaire est un alcanol en $C_1$-$C_4$ ou de l'eau.

5. Procédé selon la revendication 4, caractérisé en ce que le solvant secondaire est du méthanol, de l'éthanol ou de l'isopropanol.

6. Procédé selon l'une quelconque des revendications 1 à 5, caractérisé en ce que le solvant primaire est de l'acide chlorhydrique et le solvant secondaire est du méthanol, de l'éthanol ou de l'eau.

7. Procédé selon l'une quelconque des revendications 1 à 6, caractérisé en ce que l'on ajoute le solvant secondaire à une température d'environ 20 °C à environ 35 °C.

8. Procédé selon l'une quelconque des revendications 1 à 7, caractérisé en ce que la vitesse d'addition du solvant secondaire à la solution est d'environ 1 g/min à environ 1600 kg/min.

9. Procédé selon la revendication 8, caractérisé en ce que la vitesse d'addition du solvant secondaire à la solution est d'environ 100 g/min à environ 800 kg/min.

10. Procédé selon l'une quelconque des revendications 1 à 9, caractérisé en ce que la durée de l'addition du solvant secondaire est d'environ 5 secondes à environ 30 minutes.

**11.** Procédé selon la revendication 10, caractérisé en ce que la durée de l'addition du solvant secondaire est d'environ 1 minute à environ 20 minutes.